# EUROPEAN PATENT APPLICATION

(11) **EP 4 378 900 A1**
(43) Date of publication of application: **05.06.2024**
(21) Application number: 22210444.0
(22) Date of filing: 30.11.2022
(51) Int. Cl.: C02F 3/28, C02F 3/34, C02F 1/66, C02F 103/34, C02F 103/36, C02F 1/26, C02F 101/34

(54) **PROCESS AND PLANT FOR TREATMENT OF WASTEWATER STREAM FROM ACRYLIC ACID AND/OR ACRYLATE PRODUCTION PLANT**

(71) Applicant: Lummus Technology LLC, Houston, TX 77086 (US)
(72) Inventor: MITTELSTÄDT, Sabine, 60439 Frankfurt am Main (DE); GUTERMUTH, Thomas, 60439 Frankfurt am Main (DE); KIRSTEN, Klaus, 60439 Frankfurt am Main (DE)
(74) Representative: Hoffmann Eitle

(57) **Abstract**

Provided is a process for treatment of at least one wastewater stream from an acrylic acid and/or acrylate production plant. The process for treatment comprises the steps of: (a) introducing the least one wastewater stream from the acrylic acid and/or acrylate production plant into a bioreactor (510), (b) reacting the at least one wastewater stream in the bioreactor (510) under conditions of anaerobic microbiological decomposition of undesired organic components in the presence of at least one microorganism suitable for decomposition of undesired organic components to afford methane and carbon dioxide, (c) discharging from the bioreactor (510) a treated wastewater stream depleted in undesired organic components relative to the at least one wastewater stream introduced into the bioreactor (510), and (d) incinerating a methane-and carbon dioxide-containing product gas stream discharged from the bioreactor (510), in the incineration plant (512) with an incineration air stream and an optional auxiliary fuel stream.

## Description

### TECHNICAL FIELD

The present invention relates generally to wastewater stream treatment; more specifically, the present invention relates to a process and a plant for treatment of at least one wastewater stream from an acrylic acid and/or acrylate production plant, where a bioreactor is configured for reacting the at least one wastewater stream under conditions of anaerobic microbiological decomposition of undesired organic components in the presence of at least one microorganism suitable for decomposition of undesired organic components. Typically, the content of such undesired organic components in the waste water of an acrylic acid and/or acrylates production ranges between 4 and 10 % by weight.

### BACKGROUND

Typically, wastewater from acrylic acid production plants contains several biocidal components, e.g., acrolein, formaldehyde, etc., which renders conventional biological wastewater treatment difficult and uneconomical. Thus, the occurrence of such components is undesired. Due to presence of the biocidal components in the wastewater, the wastewater is typically routed to an incineration chamber of the production plants for decomposition of organic components by burning the wastewater. Additionally, the incineration chamber of the production plants is used for purifying chemically contaminated waste gases, which are also discharged into the incineration chamber.

When burning such waste streams with contents of undesired organic components in the lower range, a large amount of energy must be supplied to heat the waste streams to a temperature at which the decomposition of the organic components occurs to a sufficient extent. Organic fuels are used as the energy source for this purpose and are also fed into the incineration chamber. Only a small part of the energy used can be recovered as heat from the waste gas, and is routinely used to generate steam. Nevertheless, a significant portion of the heat quantity remains unused. Fossil fuel is used to generate this unused heat, which increases operating costs and also leads to the undesirable formation of carbon dioxide (CO₂), which is released into the environment.

Therefore, there is a need to address aforementioned technical drawbacks in existing known technologies in treating wastewater stream from acrylic acid and/or acrylate production plants.

### SUMMARY

The present invention seeks to provide an improved approach for treating at least one wastewater stream from an acrylic acid and/or acrylate production plant. An aim of the present invention is to provide a solution that overcomes, at least partially, the problems encountered in the prior art and to provide a process and a plant for treatment of the at least one wastewater stream from the acrylic acid and/or acrylate production plant, where a bioreactor is configured for decomposing undesired organic components in the at least one wastewater stream, under anaerobic conditions, to afford methane, carbon dioxide and treated wastewater. A methane- and carbon dioxide-containing product gas stream from the bioreactor is introduced into an incineration plant without an intermediate purification step, thereby significantly reducing the incinerator's need for fossil fuels in addition to substituting a part of the remaining fuel requirement of the incineration plant, and, at the same time, reducing the carbon dioxide emission of the plant. The object of the present invention is achieved by the solutions provided in the enclosed independent claims. Advantageous implementations of the present invention are further defined in the enclosed dependent claims.

According to a first aspect, the present invention provides a process for treatment of at least one wastewater stream from an acrylic acid and/or acrylate production plant, wherein the production plant comprises at least one functional unit selected from the group of:
(1) acrylic acid synthesis stage operated by catalytic selective oxidation of a propene containing input stream with an oxygen-containing oxidant stream to obtain an acrylic acid-containing product mixture,
(2) workup stage for separation of acrylic acid from the acrylic acid-containing product mixture,
(3) esterification stage for esterification of acrylic acid with at least one alcohol,

wherein the at least one wastewater stream contains undesired organic components, in particular at least one compound selected from the group of:
   acrolein, further aldehydes, for example formaldehyde; formic acid, further organic acids, for example acetic acid; ethers,
wherein the process for treatment comprises the steps of:
   (a) providing the least one wastewater stream from the acrylic acid and/or acrylate production plant,
   (b) introducing the least one wastewater stream into a bioreactor,
   (c) reacting the at least one wastewater stream in the bioreactor under conditions of anaerobic microbiological decomposition of undesired organic components in the presence of at least one microorganism suitable for decomposition of undesired organic components to afford methane and carbon dioxide,
   (d) discharging from the bioreactor a treated wastewater stream depleted in undesired organic components relative to the at least one wastewater stream introduced into the bioreactor,
   (e) discharging a methane- and carbon dioxide-containing product gas stream from the bioreactor, and
   (f) introducing the methane- and carbon dioxide-containing product gas stream into an incineration plant, incinerating the methane- and carbon dioxide-containing product gas stream in the incineration plant with an incineration air stream and an optional auxiliary fuel stream, discharging a flue gas stream from the incineration plant.

The process for treatment of the at least one wastewater stream from the acrylic acid and/or acrylate production plant is of advantage in that the process enables to degrade the undesired organic components in the at least one wastewater stream by reacting the at least one wastewater stream in the bioreactor under conditions of anaerobic microbiological decomposition to afford methane, carbon dioxide and treated wastewater. The methane- and carbon dioxide-containing product gas stream from the bioreactor is advantageously used in the incineration plant without an intermediate purification step to substitute a part of fuel requirement of the incineration plant, thereby significantly reducing the incinerator's need for fossil fuels which in turn reduces operating costs of the production plants. At the same time, the carbon dioxide emissions of the plants are reduced. The treated wastewater may be discharged directly into an open body of water or treated further in one or more process steps beforehand.

According to a second aspect, the present invention provides a plant for treatment of at least one wastewater stream from an acrylic acid and/or acrylate production plant, wherein the production plant comprises at least one functional unit selected from the group of:
(1) acrylic acid synthesis stage operated by catalytic selective oxidation of a propene containing input stream with an oxygen-containing oxidant stream to obtain an acrylic acid-containing product mixture,
(2) workup stage for separation of acrylic acid from the acrylic acid-containing product mixture,
(3) esterification stage for esterification of acrylic acid with at least one alcohol, wherein the plant comprises the following further assemblies and functional units in fluid connection with one another:
   (a) means for providing the at least one wastewater stream from the acrylic acid and/or acrylate production plant, wherein the at least one wastewater stream contains undesired organic components,
   (b) a bioreactor configured for reacting the at least one wastewater stream under conditions of anaerobic microbiological decomposition of undesired organic components in the presence of at least one microorganism suitable for decomposition of undesired organic components to afford methane and carbon dioxide, means for introducing the at least one wastewater stream into the bioreactor,
   (c) means for discharging from the bioreactor a treated wastewater stream, wherein the treated wastewater stream is depleted in undesired organic components relative to the at least one wastewater stream introduced into the bioreactor,
   (e) means for discharging a methane- and carbon dioxide-containing product gas stream from the bioreactor, and
   (f) an incineration plant, means for introducing the methane- and carbon dioxide-containing product gas stream into the incineration plant, means for introducing an incineration air stream and an optional auxiliary fuel stream into the incineration plant, means for discharging a flue gas stream from the incineration plant.

The plant for treatment of the at least one wastewater stream from the acrylic acid and/or acrylate production plant is of advantage in that the bioreactor of the plant enables to degrade the undesired organic components in the at least one wastewater stream by reacting the at least one wastewater stream in the bioreactor under conditions of anaerobic microbiological decomposition to afford methane, carbon dioxide and treated wastewater. The methane- and carbon dioxide-containing product gas stream from the bioreactor is advantageously used in the incineration plant without an intermediate purification step to substitute a part of fuel requirement of the incineration plant, thereby significantly reducing the incinerator's need for fossil fuels which in turn reduces operating costs of the production plants. At the same time, the carbon dioxide emissions of the plants are reduced. The treated wastewater may be discharged directly into an open body of water or treated further in one or more process steps beforehand.

Embodiments of the present invention eliminate the aforementioned drawbacks in existing known approaches in treating the at least one wastewater stream from the acrylic acid and/or acrylate production plant by integrating the bioreactor to the production plant for decomposing the undesired organic components under conditions of anaerobic microbiological decomposition and utilizing the methane- and carbon dioxide-containing product gas stream from the bioreactor to substitute the part of fuel requirement of the incineration plant in order to reduce the incinerator's need for fossil fuels, and in order to reduce the carbon dioxide emission of the plant.

Additional aspects, advantages, features and objects of the present invention are made apparent from the drawings and the detailed description of the illustrative embodiments construed in conjunction with the appended claims that follow. It will be appreciated that features of the present invention are susceptible to being combined in various combinations without departing from the scope of the present invention as defined by the appended claims.

### BRIEF DESCRIPTION OF THE DRAWINGS

The summary above, as well as the following detailed description of illustrative embodiments, is better understood when read in conjunction with the appended drawings. To illustrate the present invention, exemplary constructions of the invention are shown in the drawings. However, the present invention is not limited to specific methods and instrumentalities disclosed herein. Moreover, those in the art will understand that the drawings are not to scale. Wherever possible, the same elements have been indicated by identical numbers. Embodiments of the present invention will now be described, by way of example only, with reference to the following diagrams wherein:
FIG. 1 is a schematic diagram of a prior art acrylic acid synthesis stage for producing an acrylic acid-containing product mixture;
FIG. 2 is a schematic diagram of a prior art workup stage for separating of acrylic acid from an acrylic acid-containing product mixture;
FIG. 3 is a schematic diagram of a prior art esterification stage for esterification of acrylic acid with at least one alcohol;
FIG. 4 is a block diagram of a prior art plant for producing acrylic acid and/or acrylate with wastewater stream; and
FIG. 5 is a block diagram of a plant for treatment of at least one wastewater stream from an acrylic acid and/or acrylate production plant according to an embodiment of the present invention.

### DETAILED DESCRIPTION OF EMBODIMENTS

The following detailed description illustrates embodiments of the present invention and ways in which they can be implemented. Although some modes of carrying out the present invention have been disclosed, those skilled in the art would recognize that other embodiments for carrying out or practicing the present invention are also possible.

As used herein, several terms are defined below:
The conditions of anaerobic microbiological decomposition or aerobic microbiological decomposition are known to those skilled in the art from the prior art. These are the physicochemical conditions in the respective bioreactors, comprising temperature, pressure, pH, and flowrates of reactants and products, under which a measurable, preferably industrially relevant, conversion of hydrocarbons to anaerobic or aerobic microbiological decomposition is achieved.

The conditions of acrylic acid synthesis stage, workup stage, and esterification stage of the acrylic acid and/or acrylate production plant are known to those skilled in the art from the prior art. These are the physicochemical conditions in the respective reactors/columns, comprising temperature, pressure, pH, flowrates of reactants and products.

Undesired organic components are to be understood as components that form unintentionally in the course of the synthesis of acrylic acid and/or acrylates, either as byproducts or degradation products of the chemical reactions occurring, or during workup or further processing of intermediate products or end products of the syntheses. Examples of such undesired organic components are acrolein, further aldehydes, for example formaldehyde; formic acid, further organic acids, for example acetic acid; ethers. Typically, the content of such undesired organic components in the waste water of an acrylic acid and/or acrylates production ranges between 4 and 10 % by weight.

Pressures, if any, are reported in absolute pressure units, bar(a) for short, or in gauge pressure units, bar(g) for short, unless otherwise stated in the particular individual context.

A fluid connection between two regions of the apparatus or plant according to the invention is to be understood as meaning any type of connection whatsoever which makes it possible for a fluid, for example a gas stream, to flow from one to the other of the two regions, neglecting any interposed regions or components. In particular a direct fluid connection is to be understood as meaning any type of connection whatsoever which makes it possible for a fluid, for example a gas stream, to flow directly from one to the other of the two regions, wherein no further regions or components are interposed with the exception of purely transportational operations and the means required therefor, for example pipelines, valves, pumps, compressors, reservoirs. One example would be a pipeline leading directly from one to the other of the two regions.

A means is to be understood as meaning something that enables or is helpful in the achievement of a goal. In particular, means for performing a particular process step are to be understood as meaning any physical articles that would be considered by a person skilled in the art in order to be able to perform this process step. For example, a person skilled in the art will consider means of introducing or discharging a material stream to include any transporting and conveying apparatuses, i.e., for example pipelines, pumps, compressors, valves, which seem necessary or sensible to said skilled person for performance of this process step on the basis of his knowledge of the art.

For the purposes of this description steam is to be understood as being synonymous with water vapor unless the opposite is indicated in an individual case. By contrast, the term "water" refers to water in the liquid state of matter unless otherwise stated in an individual case.

Optional or optionally means that the subsequently described event or circumstances may or may not occur. The description includes instances where the event or circumstance occurs and instances where it does not occur.

"Providing" in a claim is defined to mean furnishing, supplying, making available, or preparing something. The step may be performed by any actor in the absence of express language in the claim to the contrary.

According to a first aspect, the present invention provides a process for treatment of at least one wastewater stream from an acrylic acid and/or acrylate production plant, wherein the production plant comprises at least one functional unit selected from the group of:
(1) acrylic acid synthesis stage operated by catalytic selective oxidation of a propene containing input stream with an oxygen-containing oxidant stream to obtain an acrylic acid-containing product mixture,
(2) workup stage for separation of acrylic acid from the acrylic acid-containing product mixture,
(3) esterification stage for esterification of acrylic acid with at least one alcohol,
   wherein the at least one wastewater stream contains undesired organic components, in particular at least one compound selected from the group of:
      acrolein, further aldehydes, for example formaldehyde; formic acid, further organic acids, for example acetic acid; ethers,
   wherein the process for treatment comprises the steps of:
      (a) providing the least one wastewater stream from the acrylic acid and/or acrylate production plant,
      (b) introducing the least one wastewater stream into a bioreactor,
      (c) reacting the at least one wastewater stream in the bioreactor under conditions of anaerobic microbiological decomposition of undesired organic components in the presence of at least one microorganism suitable for decomposition of undesired organic components to afford methane and carbon dioxide,
      (d) discharging from the bioreactor a treated wastewater stream depleted in undesired organic components relative to the at least one wastewater stream introduced into the bioreactor,
      (e) discharging a methane- and carbon dioxide-containing product gas stream from the bioreactor, and
      (f) introducing the methane- and carbon dioxide-containing product gas stream into an incineration plant, incinerating the methane- and carbon dioxide-containing product gas stream in the incineration plant with an incineration air stream and an optional auxiliary fuel stream, discharging a flue gas stream from the incineration plant.

The process for treatment of the at least one wastewater stream from the acrylic acid and/or acrylate production plant is of advantage in that the process enables to degrade the undesired organic components in the at least one wastewater stream by reacting the at least one wastewater stream in the bioreactor under conditions of anaerobic microbiological decomposition to afford methane, carbon dioxide and treated wastewater. The methane- and carbon dioxide-containing product gas stream from the bioreactor is advantageously used in the incineration plant without an intermediate purification step to substitute a part of fuel requirement of the incineration plant, thereby significantly reducing the incinerator's need for fossil fuels which in turn reduces operating costs of the production plants. At the same time, the carbon dioxide emissions of the plants are reduced. Advantageously, the process of the present invention may achieve wastewater qualities that meet all the usual regulations.

Optionally, the acrylic acid-containing product mixture is produced by a two-stage reactor that comprises a first stage reactor and a second stage reactor.

Optionally, the treated wastewater stream obtained in step (d) is sent to a further treatment step, purification step or disposal step. Optionally, the treated wastewater stream obtained in step (d) is sent directly to a locally available municipal or industrial wastewater treatment plant or, after one or more further treatment steps, discharged into open waters.

Optionally, within the acrylic acid and/or acrylate production plant, at least one further waste stream or byproduct stream is obtained. The at least one further waste stream or by-product stream comprises at least one element selected from the group of: offgas stream, wastewater stream, solid waste stream, melts-comprising waste stream. The at least one further waste stream or byproduct stream is also sent to the incineration plant. Optionally, the least one further waste stream or byproduct stream is incinerated in the incineration plant using the methane- and carbon dioxide-containing product gas stream from the bioreactor.

Optionally, the at least one wastewater stream from the acrylic acid and/or acrylate production plant is initially introduced into a buffer vessel and subsequently introduced into the bioreactor from the buffer vessel. Accordingly, the process of the present invention makes a composition of the at least one wastewater stream uniform and reduces possible fluctuations in the concentration of the undesired organic components.

Optionally, before introduction into the bioreactor the at least one wastewater stream from the acrylic acid and/or acrylate production plant is admixed with at least one additive, wherein the at least one additive is selected from a group comprising:
water; aqueous acid solution, preferably aqueous HCI solution, aqueous alkali metal hydroxide solution, preferably NaOH solution; chelate formers, preferably EDTA; nutritional supplements for microorganisms, preferably inorganic or mineral nutritional supplements, most preferably nutritional supplements containing Na, K, Ca, P, SO₄²⁻.

Optionally, the reacting of the at least one wastewater stream in the bioreactor is carried out under conditions of anaerobic microbiological decomposition of undesired organic components in the presence of at least one microorganism of the genus *Methanobacterium.*

Optionally, at least a portion of the treated wastewater stream discharged from the bioreactor is recycled to the inlet of the bioreactor as a dilution stream. Accordingly, the dilution stream enables to reduce and control the concentration of the undesired organic components at an input to the bioreactor. Further, the dilution stream reduces possible fluctuations in the concentration of the undesired organic components in the bioreactor. Optionally, before introduction into the incineration plant the methane- and carbon dioxide-containing product gas stream is compressed using a compressor to a pressure between 5 mbar(g) and 10 bar(g), or preferably between 50 mbar(g) and 2 bar(g), most preferably of 100 mbar(g) to 1 bar(g).

Optionally, using the flue gas stream discharged from the incineration plant, through indirect heat exchange in at least one heat exchanger,
at least one first material stream used in the acrylic acid and/or acrylate production plant is heated, and/or
at least one first steam stream is generated which is at least partially used for driving a compressor and/or for generating electrical energy. Accordingly, the overall energy and/or heat balance of the process can be further improved.

Optionally, the at least one first material stream in the acrylic acid and/or acrylate production plant is selected from a group comprising the elements:
propene containing input stream, oxygen-containing oxidant stream, at least one water stream and/or steam stream used for heating at least one distillation column present in the workup stage for separation of acrylic acid from the acrylic acid-containing product mixture.

Optionally, the heat liberated during the reacting of the at least one wastewater stream in the bioreactor under conditions of anaerobic microbiological decomposition of undesired organic components is at least partially used for preheating the at least one wastewater stream before said stream is introduced into the bioreactor. Accordingly, the overall energy and/or heat heat balance of the process can be further improved.

Optionally, the at least one wastewater stream is sent to at least one further purification step so that the concentration of the undesired organic components in the wastewater stream reaches or falls below a specified threshold value.

Optionally, the at least one further purification step comprises a treatment step under conditions of aerobic microbiological decomposition of undesired organic components.

According to a second aspect, the present invention provides a plant for treatment of at least one wastewater stream from an acrylic acid and/or acrylate production plant, wherein the production plant comprises at least one functional unit selected from the group of:
(1) acrylic acid synthesis stage operated by catalytic selective oxidation of a propene containing input stream with an oxygen-containing oxidant stream to obtain an acrylic acid-containing product mixture,
(2) workup stage for separation of acrylic acid from the acrylic acid-containing product mixture,
(3) esterification stage for esterification of acrylic acid with at least one alcohol,
wherein the plant comprises the following further assemblies and functional units in fluid connection with one another:
(a) means for providing the at least one wastewater stream from the acrylic acid and/or acrylate production plant, wherein the at least one wastewater stream contains undesired organic components,
(b) a bioreactor configured for reacting the at least one wastewater stream under conditions of anaerobic microbiological decomposition of undesired organic components in the presence of at least one microorganism suitable for decomposition of undesired organic components to afford methane and carbon dioxide, means for introducing the at least one wastewater stream into the bioreactor,
(c) means for discharging from the bioreactor a treated wastewater stream, wherein the treated wastewater stream is depleted in undesired organic components relative to the at least one wastewater stream introduced into the bioreactor,
(e) means for discharging a methane- and carbon dioxide-containing product gas stream from the bioreactor, and
(f) an incineration plant, means for introducing the methane- and carbon dioxide-containing product gas stream into the incineration plant, means for introducing an incineration air stream and an optional auxiliary fuel stream into the incineration plant, means for discharging a flue gas stream from the incineration plant.

The plant for treatment of the at least one wastewater stream from the acrylic acid and/or acrylate production plant is of advantage in that the bioreactor of the plant enables to degrade the undesired organic components in the at least one wastewater stream by reacting the at least one wastewater stream in the bioreactor under conditions of anaerobic microbiological decomposition to afford methane, carbon dioxide and treated wastewater. The methane- and carbon dioxide-containing product gas stream from the bioreactor is advantageously used in the incineration plant without an intermediate purification step to substitute a part of fuel requirement of the incineration plant, thereby significantly reducing the incinerator's need for fossil fuels which in turn reduces operating costs of the production plants. At the same time, the carbon dioxide emissions of the plants are reduced. The treated wastewater may be discharged directly into an open body of water or treated further in one or more process steps beforehand. Advantageously, the plant of the present invention may achieve wastewater qualities that meet all the usual regulations.

Optionally, plant is configured and/or means are provided such that using the flue gas stream discharged from the incineration plant, through indirect heat exchange in at least one heat exchanger,
at least one first material stream used in the acrylic acid and/or acrylate production plant is heated and/or
at least one first steam stream is generated which is at least partially used for driving a compressor and/or for generating electrical energy.

Embodiments of the present invention substantially eliminate or at least partially address the aforementioned technical drawbacks in existing technologies in treating the at least one wastewater stream from the acrylic acid and/or acrylate production plant by integrating the bioreactor to the production plant for decomposing the undesired organic components under conditions of anaerobic microbiological decomposition.

### DETAILED DESCRIPTION OF THE DRAWINGS

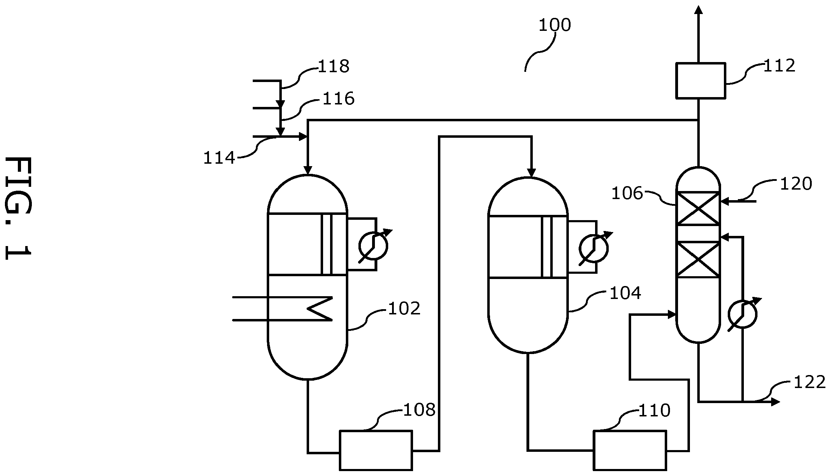

**FIG. 1** is a schematic diagram of a prior art acrylic acid synthesis stage **100** for producing an acrylic acid-containing product mixture. The acrylic acid synthesis stage **100** comprises a first stage reactor **102,** a second stage reactor **104,** an absorbing column **106,** a first waste-heat boiling unit **108,** a second waste-heat boiling unit **110,** and an incinerator plant **112.** The first stage reactor **102** is configured for catalytically oxidizing a propene containing input stream **114** along with an oxygen-containing oxidant stream **116** and steam **118** into an acrolein containing gas stream. The first waste-heat boiling unit **108** is configured for utilizing heat from the acrolein containing gas stream to generate hot water stream or steam. The second stage reactor **104** is configured for catalytically oxidizing the acrolein containing gas stream into an acrylic acid-containing gas stream. The second waste-heat boiling unit **110** is configured for utilizing heat from the acrylic acid-containing gas stream to generate hot water stream or steam. The acrylic acid-containing gas stream is sent to the absorbing column **106** where acrylic acid is absorbed in water **120** to form the acrylic acid-containing product mixture. The acrylic acid-containing product mixture is sent to further purification steps **122.** Upstream waste gas is sent to the incinerator plant **112** for incinerating the waste gas. At least a portion of the waste gas is recycled back to the first stage reactor 102.

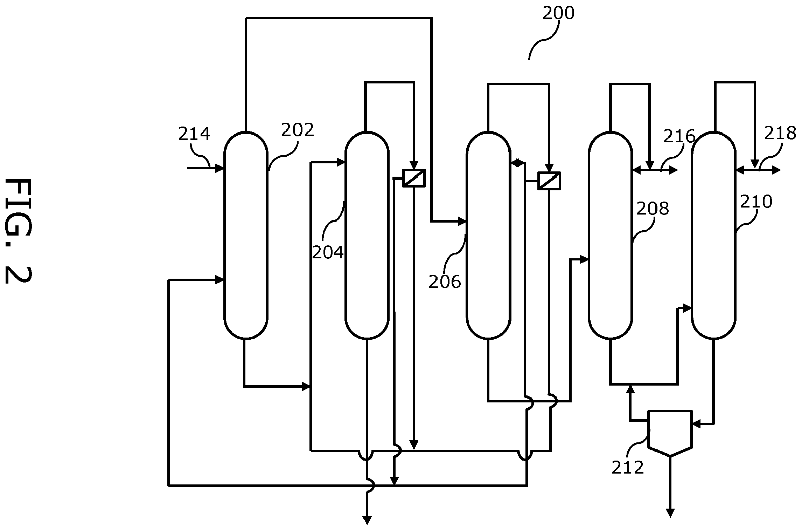

**FIG. 2** is a schematic diagram of a prior art workup stage **200** for separating of acrylic acid from an acrylic acid-containing product mixture **214.** The workup stage **200** comprises an extraction column **202,** a raffinate-stripping column **204,** a solvent separation column **206,** a light-ends cut column **208,** an acrylic acid product column **210,** and a decomposition evaporator **212.** The acrylic acid-containing product mixture **214** from an acrylic acid synthesis stage is provided to the extraction column **202.** The extraction column **202** is configured for separating acrylic acid from the acrylic acid-containing product mixture by liquid-liquid extraction with a solvent and obtaining an extractant stream containing acrylic acid and acetic acid as a top product. A raffinate stream from the extraction column **202** is provided to the raffinate-stripping column **204** for stripping the solvent from the raffinate stream. The extractant stream is provided to the solvent separation column **206** for separating the solvent from the extractant stream and obtaining a crude acrylic acid mixture as a bottom product. A two-phase overhead stream from the solvent separation column **206** is separated into an organic phase containing the solvent which recycled to the extraction column **202,** and an aqueous phase which is mixed with the raffinate stream. The crude acrylic acid mixture is provided to the light-ends cut column **208** for separating acetic acid **216** and/or light-ends from the crude acrylic acid mixture and obtaining an acrylic acid containing stream as a bottom product. The Light ends are the light hydrocarbon gases and liquids. The acrylic acid containing stream is provided to the acrylic acid product column **210** for purifying the acrylic acid containing stream to obtain purified acrylic acid **218** as an overhead product. A bottom stream containing acrylic acid dimers from the acrylic acid product column **210,** is provided to the decomposition evaporator **212** for decomposing acrylic acid dimers into monomers. The acrylic acid monomers from the decomposition evaporator **212** are recycled to the acrylic acid product column **210.** The waste oil is obtained as a bottom product from the decomposition evaporator **212,** which is further incinerated.

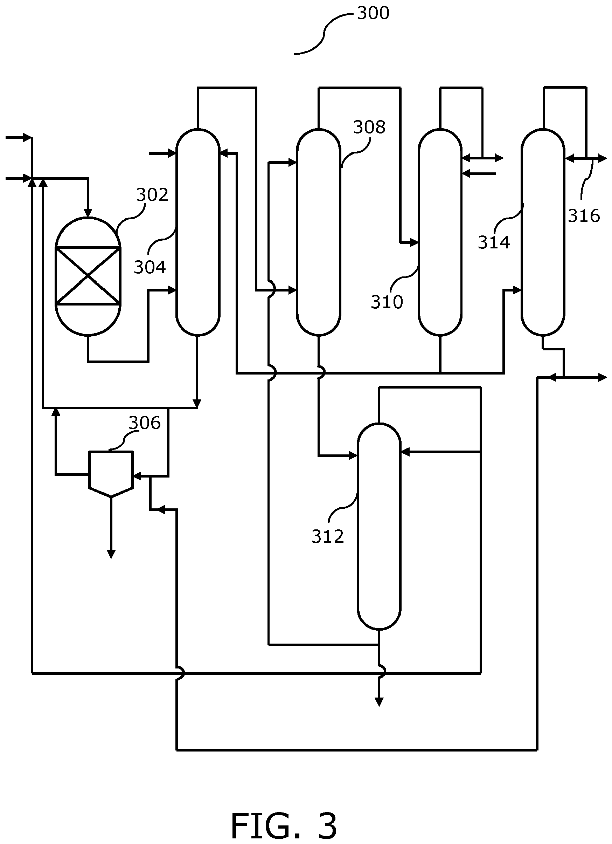

**FIG. 3** is a schematic diagram of a prior art esterification stage **300** for esterification of acrylic acid with at least one alcohol. The esterification stage **300** comprises an esterification reactor **302,** an ester stripping column **304,** a bottom stripper **306,** an alcohol extraction column **308,** a light-ends cut column **310,** an alcohol recovery column **312,** and an acrylate product column **314.** The esterification reactor **302** is configured for reacting the acrylic acid from a workup stage with an alcohol feedstock using a strong acid catalyst. Effluents from the esterification reactor **302** is provided to the ester stripping column **304** for separating unreacted acrylic acid as a bottom product which is recycled to the esterification reactor **302.** A first crude acrylates containing product mixture is obtained as an overhead product from the ester stripping column **304.** Optionally, the bottom stripper **306** is configured for decomposing a portion of the unreacted acrylic acid to obtain acrylic acid monomers which are recycled to the esterification reactor **302.** The first crude acrylates containing product mixture is provided to the alcohol extraction column **308** for extracting alcohol from the first crude acrylates containing product mixture. A bottom product containing alcohol is provided to the alcohol recovery column **312** for recovering alcohol from the bottom product as an overhead product and recycling the recovered alcohol to the esterification reactor **302.** A portion of a bottom product from the alcohol recovery column **312** is provided to the alcohol extraction column **308** for extracting alcohol. An overhead product from the alcohol extraction column **308** is provided to the light-ends cut column **310** for separating light-ends from the overhead product. A bottom product from the light-ends cut column **310** is provided to the acrylate product column **314** for separating high boiling components from the bottom product and obtaining a final product containing purified acrylates **316.**

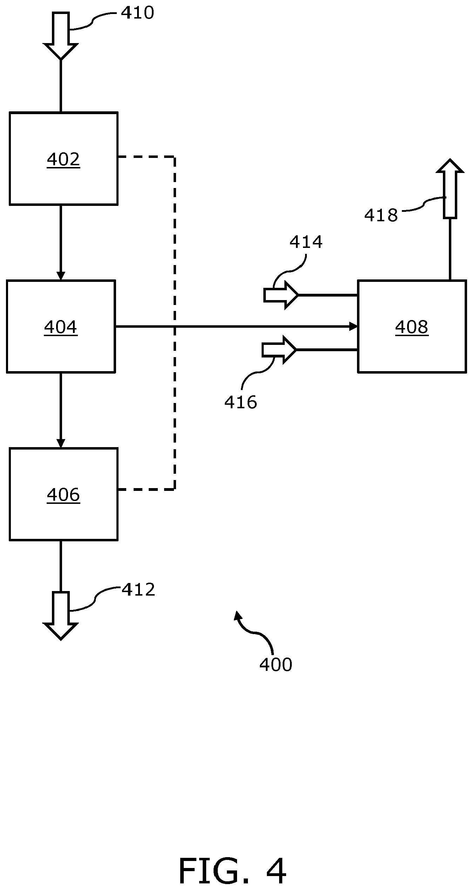

**FIG. 4** is a block diagram of a prior art plant **400** for producing acrylic acid and/or acrylate with wastewater stream. The plant **400** comprises at least one functional unit selected from the group of: an acrylic acid synthesis stage **402,** the workup stage **404,** and the esterification stage **406,** and an incineration plant **408.** The acrylic acid synthesis stage **402** is configured for operating by catalytic selective oxidation of a propene containing input stream with an oxygen-containing oxidant stream **410** to obtain an acrylic acid-containing product mixture. The workup stage **404** is configured for separation of acrylic acid from the acrylic acid-containing product mixture. The esterification stage **406** is configured for esterification of acrylic acid with at least one alcohol to obtain acrylates containing product **412.** The incineration plant **408** is configured for incinerating at least one waste water stream and/or waste gas stream from acrylic acid synthesis stage **402,** and/or the workup stage **404,** and/or the esterification stage **406,** with an incineration air stream **414** and an optional auxiliary fuel stream **416** and discharging flue gas stream **418** from the incineration plant **408.**

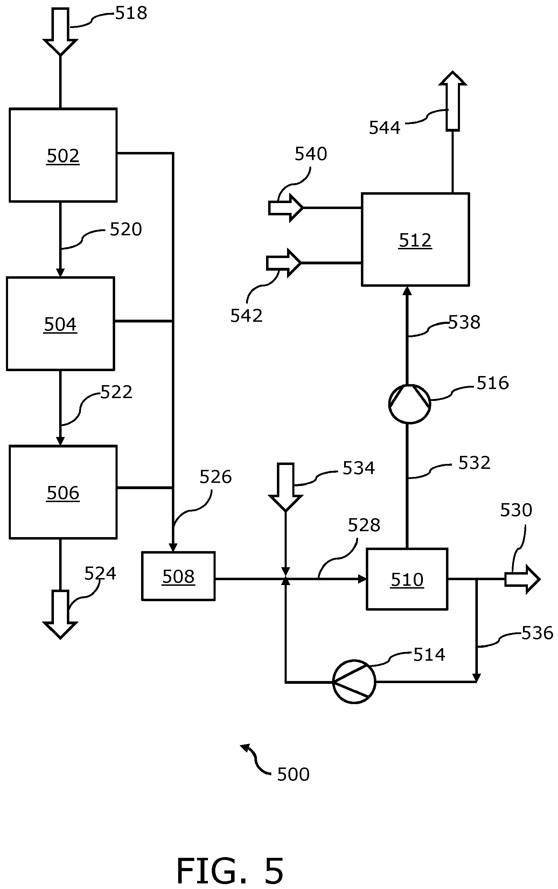

**FIG. 5** is a block diagram of a plant **500** for treatment of at least one wastewater stream from an acrylic acid and/or acrylate production plant according to an embodiment of the present invention. The acrylic acid and/or acrylate production plant comprises an acrylic acid synthesis stage **502,** a workup stage **504,** and an esterification stage **506.** The plant **500** comprises the following further assemblies and functional units in fluid connection with one another: a buffer vessel **508,** a bioreactor **510,** an incineration plant **512,** a recycle pump **514,** a compressor **516,** an input stream providing means **518,** an acrylic acid-containing product mixture discharging means **520,** an acrylic acid discharging means **522,** an acrylates containing product discharging means **524,** a wastewater stream providing means **526,** a wastewater stream introducing means **528,** a treated wastewater discharging means **530,** a product gas stream discharging means **532,** an additive introducing means **534,** a diluent stream providing means **536,** a product gas stream introducing means **538,** an incineration air stream introducing means **540,** an auxiliary fuel stream introducing means **542,** and a flue gas discharging means **544.**

The input stream providing means **518** is configured for providing the propene containing input stream, and an oxygen-containing oxidant stream to the acrylic acid synthesis stage **502.** The acrylic acid synthesis stage **502** is configured for operating by catalytic selective oxidation of the propene containing input stream with the oxygen-containing oxidant stream to obtain an acrylic acid-containing product mixture and discharging the acrylic acid-containing product mixture through the acrylic acid-containing product mixture discharging means **520.** The workup stage **504** is configured for separating acrylic acid from the acrylic acid-containing product mixture and discharging the acrylic acid through the acrylic acid discharging means **522.** The esterification stage **506** is configured for esterifying acrylic acid with at least one alcohol and discharging acrylates containing product through the acrylates containing product discharging means **524.**

Although, in the example shown in Fig. 5, all of the units **502, 504, 506** are in fluid connection with the wastewater stream providing means **526,** the invention relates also to examples not shown in the figures in which any one of the units **502, 504, 506** alone are in fluid connection with the wastewater stream providing means **526,** or with any combination of two of the units **502, 504, 506** are in fluid connection with the wastewater stream providing means **526.**

The wastewater stream providing means **526** is configured for providing the at least one wastewater stream from the acrylic acid synthesis stage and/or the workup stage **504** and/or the esterification stage **506.** The at least one wastewater stream from the acrylic acid and/or acrylate production plant is initially introduced into the buffer vessel **508** and subsequently introduced into the bioreactor **510** from the buffer vessel **508.** The bioreactor **510** is configured for receiving the at least one wastewater stream through the wastewater stream introducing means **528** and reacting the at least one wastewater stream under conditions of anaerobic microbiological decomposition of undesired organic components in the presence of at least one microorganism suitable for decomposition of undesired organic components to afford methane and carbon dioxide. Optionally, before introduction into the bioreactor **510,** the at least one wastewater stream from the acrylic acid and/or acrylate production plant is admixed with at least one additive. The at least one additive is introduced into the bioreactor **510** through the additive introducing means **534.** The treated wastewater discharging means **530** is configured for discharging, from the bioreactor **510,** a treated wastewater stream that is depleted in undesired organic components relative to the at least one wastewater stream introduced into the bioreactor **510.** Optionally, the treated wastewater stream obtained from the bioreactor **510** is sent to a further treatment step, purification step or disposal step. At least a portion of the treated wastewater stream discharged from the bioreactor **510** is recycled to an inlet of the bioreactor **510** as a dilution stream through the recycle pump **514.** The diluent stream providing means **536** is configured to provide the portion of the treated wastewater stream to the recycle pump **514.** The product gas stream discharging means **532** is configured for discharging, from the bioreactor **510,** a methane- and carbon dioxide-containing product gas stream. The incineration plant **512** is configured for receiving the methane- and carbon dioxide-containing product gas stream through the product gas stream introducing means **538** and incinerating the methane- and carbon dioxide-containing product gas stream with an incineration air stream and an optional auxiliary fuel stream. The incineration air stream is introduced to the incineration plant **512** through the incineration air stream introducing means **540.** The auxiliary fuel stream is introduced to the incineration plant **512** through the auxiliary fuel stream introducing means **542.** The flue gas discharging means **544** is configured for discharging a flue gas stream from the incineration plant **512.** Optionally, before introduction into the incineration plant **512,** the methane- and carbon dioxide-containing product gas stream is compressed using the compressor **516** to a pressure between 5 mbar(g) and 10 bar(g), or preferably between 50 mbar(g) and 2 bar(g), most preferably of 100 mbar(g) to 1 bar(g).

Modifications to embodiments of the present invention described in the foregoing are possible without departing from the scope of the present invention as defined by the accompanying claims. Expressions such as "including", "comprising", "incorporating", "have", "is" used to describe, and claim the present invention are intended to be construed in a non-exclusive manner, namely allowing for items, components or elements not explicitly described also to be present. Reference to the singular is also to be construed to relate to the plural.

### LIST OF REFERENCE NUMERALS

100 - acrylic acid synthesis stage
102 - first stage reactor
104 - second stage reactor
106 - absorbing column
108 - first waste-heat boiling unit
110 - second waste-heat boiling unit
112 - incinerator plant
114 - propene containing input stream
116 - oxygen-containing oxidant stream
118 - steam
120 - water
122 - further purification steps in acrylic acid synthesis stage
200 - workup stage
202 - extraction column
204 - raffinate-stripping column
206 - solvent separation column
208- light-ends cut column
210 - acrylic acid product column
212 - decomposition evaporator
214 - acrylic acid-containing product mixture
216 - acetic acid
218 - purified acrylic acid
300 - esterification stage
302 - an esterification reactor
304 - ester stripping column
306 - bottom stripper
308 - alcohol extraction column
310 - light-ends cut column
312 - alcohol recovery column
314 - acrylate product column
316 - purified acrylates
400 - plant
402 - acrylic acid synthesis stage
404 - workup stage
406 - esterification stage
408 - incineration plant
410 - propene containing input stream with an oxygen-containing oxidant stream
412 - acrylates containing product
414 - incineration air stream
416 - optional auxiliary fuel stream
418 - flue gas stream
500 - plant
502 - acrylic acid synthesis stage
504 - workup stage
506 - esterification stage
508 - buffer vessel
510 - bioreactor
512 - incineration plant
514 - recycle pump
516 - compressor
518 - input stream providing means
520 - acrylic acid-containing product mixture discharging means
522 - acrylic acid discharging means
524 - acrylates containing product discharging means
526 - wastewater stream providing means
528 - wastewater stream introducing means
530 - treated wastewater discharging means
532 - product gas stream discharging means
534 - additive introducing means
536 - diluent stream providing means
538 - product gas stream introducing means
540 - incineration air stream introducing means
542 - auxiliary fuel stream introducing means
544 - flue gas discharging means

## Claims

1. Process for treatment of at least one wastewater stream from an acrylic acid and/or acrylate production plant, wherein the production plant comprises at least one functional unit selected from the group of:
(1) acrylic acid synthesis stage (502) operated by catalytic selective oxidation of a propene containing input stream with an oxygen-containing oxidant stream to obtain an acrylic acid-containing product mixture,
(2) workup stage (504) for separation of acrylic acid from the acrylic acid-containing product mixture,
(3) esterification stage (506) for esterification of acrylic acid with at least one alcohol, wherein the at least one wastewater stream contains undesired organic components, in particular at least one compound selected from the group of:
acrolein, further aldehydes, for example formaldehyde; formic acid, further organic acids, for example acetic acid; ethers,
wherein the process for treatment comprises the steps of:
(a) providing the least one wastewater stream from the acrylic acid and/or acrylate production plant,
(b) introducing the least one wastewater stream into a bioreactor (510),
(c) reacting the at least one wastewater stream in the bioreactor (510) under conditions of anaerobic microbiological decomposition of undesired organic components in the presence of at least one microorganism suitable for decomposition of undesired organic components to afford methane and carbon dioxide,
(d) discharging from the bioreactor (510) a treated wastewater stream depleted in undesired organic components relative to the at least one wastewater stream introduced into the bioreactor (510),
(e) discharging a methane- and carbon dioxide-containing product gas stream from the bioreactor (510), and
(f) introducing the methane- and carbon dioxide-containing product gas stream into an incineration plant (512), incinerating the methane-and carbon dioxide-containing product gas stream in the incineration plant (512) with an incineration air stream and an optional auxiliary fuel stream, discharging a flue gas stream from the incineration plant (512).

2. Process according to Claim 1, **characterized in that** the treated wastewater stream obtained in step (d) is sent to a further treatment step, purification step or disposal step.

3. Process according to Claim 1 or 2, **characterized in that** within the acrylic acid and/or acrylate production plant at least one further waste stream or byproduct stream is obtained, wherein the at least one further waste stream or byproduct stream comprises at least one element selected from the group of:
off-gas stream, wastewater stream, solid waste stream, melts-comprising waste stream, wherein the at least one further waste stream or byproduct stream is also sent to the incineration plant (512).

4. Process according to any of the preceding claims, **characterized in that** the at least one wastewater stream from the acrylic acid and/or acrylate production plant is initially introduced into a buffer vessel (508) and subsequently introduced into the bioreactor (510) from the buffer vessel (508).

5. Process according to any of the preceding claims, **characterized in that** before introduction into the bioreactor (510) the at least one wastewater stream from the acrylic acid and/or acrylate production plant is admixed with at least one additive, wherein the at least one additive is selected from a group comprising:
water; aqueous acid solution, preferably aqueous HCI solution, aqueous alkali metal hydroxide solution, preferably NaOH solution; chelate formers, preferably EDTA; nutritional supplements for microorganisms, preferably inorganic or mineral nutritional supplements, most preferably nutritional supplements containing Na, K, Ca, P, SO₄²⁻.

6. Process according to any of the preceding claims, **characterized in that** the reacting of the at least one wastewater stream in the bioreactor (510) is carried out under conditions of anaerobic microbiological decomposition of undesired organic components in the presence of at least one microorganism of the genus *Methanobacterium.*

7. Process according to any of the preceding claims, **characterized in that** at least a portion of the treated wastewater stream discharged from the bioreactor (510) is recycled to the inlet of the bioreactor (510) as a dilution stream.

8. Process according to any of the preceding claims, **characterized in that** before introduction into the incineration plant (512) the methane- and carbon dioxide-containing product gas stream is compressed using a compressor (516) to a pressure between 5 mbar(g) and 10 bar(g), or preferably between 50 mbar(g) and 2 bar(g), most preferably of 100 mbar(g) to 1 bar(g).

9. Process according to any of the preceding claims, **characterized in that** using the flue gas stream discharged from the incineration plant (512), through indirect heat exchange in at least one heat exchanger,
at least one first material stream used in the acrylic acid and/or acrylate production plant is heated, and/or
at least one first steam stream is generated which is at least partially used for driving a compressor (516) and/or for generating electrical energy.

10. Process according to claim 9, **characterized in that** the at least one first material stream in the acrylic acid and/or acrylate production plant is selected from a group comprising the elements:
propene containing input stream, oxygen-containing oxidant stream, at least one water stream and/or steam stream used for heating at least one distillation column present in the workup stage (504) for separation of acrylic acid from the acrylic acid-containing product mixture.

11. Process according to any of the preceding claims, **characterized in that** the heat liberated during the reacting of the at least one wastewater stream in the bioreactor (510) under conditions of anaerobic microbiological decomposition of undesired organic components is at least partially used for preheating the at least one wastewater stream before said stream is introduced into the bioreactor (510).

12. Process according to any of the preceding claims, **characterized in that** the at least one wastewater stream is sent to at least one further purification step so that the concentration of the undesired organic components in the wastewater stream reaches or falls below a specified threshold value.

13. Process according to any of the preceding claims, **characterized in that** the at least one further purification step comprises a treatment step under conditions of aerobic microbiological decomposition of undesired organic components.

14. Plant (500) for treatment of at least one wastewater stream from an acrylic acid and/or acrylate production plant, wherein the production plant comprises at least one functional unit selected from the group of:
(1) acrylic acid synthesis stage (502) operated by catalytic selective oxidation of a propene containing input stream with an oxygen-containing oxidant stream to obtain an acrylic acid-containing product mixture,
(2) workup stage (504) for separation of acrylic acid from the acrylic acid-containing product mixture,
(3) esterification stage (506) for esterification of acrylic acid with at least one alcohol, wherein the plant (500) comprises the following further assemblies and functional units in fluid connection with one another:
(a) means (526) for providing the at least one wastewater stream from the acrylic acid and/or acrylate production plant, wherein the at least one wastewater stream contains undesired organic components,
(b) a bioreactor (510) configured for reacting the at least one wastewater stream under conditions of anaerobic microbiological decomposition of undesired organic components in the presence of at least one microorganism suitable for decomposition of undesired organic components to afford methane and carbon dioxide, means (528) for introducing the at least one wastewater stream into the bioreactor (510),
(c) means (530) for discharging from the bioreactor (510) a treated wastewater stream, wherein the treated wastewater stream is depleted in undesired organic components relative to the at least one wastewater stream introduced into the bioreactor (510),
(e) means (532) for discharging a methane- and carbon dioxide-containing product gas stream from the bioreactor (510), and
(f) an incineration plant (512), means (538) for introducing the methane- and carbon dioxide-containing product gas stream into the incineration plant (512), means (540, 542) for introducing an incineration air stream and an optional auxiliary fuel stream into the incineration plant (512), means (544) for discharging a flue gas stream from the incineration plant (512).

15. Plant (500) according to claim 14, **characterized in that** the plant (500) is configured and/or means are provided such that using the flue gas stream discharged from the incineration plant (512), through indirect heat exchange in at least one heat exchanger,
at least one first material stream used in the acrylic acid and/or acrylate production plant is heated and/or
at least one first steam stream is generated which is at least partially used for driving a compressor (516) and/or for generating electrical energy.
